Europäisches Patentamt

European Patent Office

Office européen des brevets

(11) Numéro de publication: **0 249 563**
**A1**

## (12) DEMANDE DE BREVET EUROPEEN

(21) Numéro de dépôt: **87401332.9**

(51) Int. Cl.⁴: **A 61 K 35/54**

(22) Date de dépôt: **12.06.87**

(30) Priorité: **12.06.86 FR 8608485**

(43) Date de publication de la demande:
**16.12.87 Bulletin 87/51**

(84) Etats contractants désignés:
**AT BE CH DE ES FR GB GR IT LI LU NL SE**

(71) Demandeur: **Laumond, Gérard**
**13, Rue de la Poste**
**F-64200 Biarritz (FR)**

(72) Inventeur: **Laumond, Gérard**
**13, Rue de la Poste**
**F-64200 Biarritz (FR)**

(74) Mandataire: **Gillard, Marie-Louise et al**
**Cabinet Beau de Loménie 55, Rue d'Amsterdam**
**F-75008 Paris (FR)**

(54) **Extraits tissulaires embryonnaires d'organes animaux, utiles comme stimulants des fonctions métaboliques organiques humaines.**

(57) La présente invention a pour objet des extraits tissulaires embryonnaires d'organes animaux, administrables à l'homme, caractérisés en ce qu'ils sont obtenus par dilution ou ultrafiltration d'un extrait brut, préparé à partir d'organes, prélevés selon des techniques connues, sur des embryons avant la première moitié de la gestation et broyés dans du sérum physiologique puis filtrés.

EP 0 249 563 A1

Bundesdruckerei Berlin

## Description

Extraits tissulaires embryonnaires d'organes animaux utiles comme stimulants des fonctions métaboliques organiques humaines.

La présente invention a pour objet des extraits tissulaires embryonnaires d'organes animaux, qui peuvent être administrés à l'homme pour stimuler ses fonctions métaboliques organiques. Il s'agit d'extraits totaux et non pas de cellules embryonnaires.

Les extraits tissulaires embryonnaires d'organes animaux selon l'invention sont préparés selon différents procédés à partir d'un extrait brut soit par dilution, soit par ultrafiltration.

L'extrait brut est obtenu à partir d'organes ou de milieux organiques, prélevés selon les techniques connues, sur des embryons avant la première moitié de la gestation, par broyage dans du sérum physiologique puis filtration.

Dans la présente description, on entend par organes toute partie de l'embryon, notamment les organes pris dans leur intégralité tels que le cerveau, la rate, certaines glandes, ou en partie tels que par exemple, l'hypothalamus, le myocarde. On englobe également dans cette définition de "l'organe", le placenta. A titre d'exemple de milieu organique, on peut citer le liquide amniotique.

Lesdits organes sont prélevés sur des embryons de mammifères, tels que notamment des bovins ou ovins, avant la première moitié de la gestation. Dans ces conditions, les cellules desdits tissus ne sont pas à un stade développé de différentiation.

Le broyage est réalisé selon des moyens connus, et les conditions sont choisies pour obtenir une émulsion qui peut être éventuellement injectée. On indiquera à titre d'exemple, que de manière satisfaisante on peut broyer 0,2 cm$^3$ d'organes dans 3 cm$^3$ de sérum physiologique.

Les différents procédés d'obtention des extraits tissulaires embryonnaires selon l'invention sont décrits ci-après :

Procédé A : l'extrait brut est dilué, par exemple au 20e dans du sérum physiologique ou du plasma de Quinton en présence d'un agent conservateur, tel que le formol. L'extrait ainsi obtenu est un extrait buvable.

Procédé B : l'extrait brut est mélangé avec du sérum physiologique contenant au moins un antibiotique, ou selon une variante le broyage de l'organe est réalisé en présence d'un antibiotique. Cet extrait est un extrait injectable qui doit être conservé à l'état congelé.

Procédé C : l'extrait brut est soumis à une ultrafiltration de préférence sur un filtre de 0,22 μm. L'extrait obtenu peut être conservé à la température ambiante ou au froid (réfrigérateur). Cet extrait peut être administré par voie orale ou par injection, par exemple intramusculaire, intradermique, sous-cutanée.

Comme indiqué précédemment, les extraits tissulaires embryonnaires selon l'invention stimulent les fonctions métaboliques organiques humaines. De plus, les problèmes d'allergie peuvent être totalement évités avec les extraits selon l'invention.

Il s'agit, dans une certaine mesure, d'une révolution dans l'immunologie classique. L'homme de l'art n'ignore pas que des accidents allergiques plus ou moins graves surviennent lors d'injections chez l'homme, de tissus animaux adultes. De la même façon, l'injection de ces tissus à des races d'animaux différentes de celle du donneur entraîne des problèmes. De tels problèmes existent encore, même lorsque de telles injections sont pratiquées au sein d'une même race.

Des essais réalisés notamment chez la souris et chez l'homme ont en effet montré qu'on peut éviter les problèmes d'allergie en utilisant des tissus embryonnaires, prélevés avant la première moitié de la gestation.

On notera que pour pouvoir être injectés, les extraits tissulaires doivent présenter une stérilité absolue. Celle-ci est obtenue d'une part, par les techniques de prélèvements vétérinaires et de dissection classique en chambre stérile, sous hotte à flux laminaire et, d'autre part, par le procédé d'obtention.

Par exemple, selon le procédé B, on utilise au moins un antibiotique approprié. Dans ce cas, le broyage de l'organe est avantageusement réalisé au sein d'une solution isotonique de NaCl, 9 g par litre en présence d'au moins un antibiotique. Le ou les antibiotiques sont choisis en fonction de leurs propriétés non allergisantes et de leur spectre d'action tant sur les germes susceptibles d'infecter la préparation que sur les zoonoses éventuellement transmissibles à l'homme. Dans la présente description, on entend par antibiotique approprié, tout antibiotique ou mélange d'antibiotiques ayant de telles propriétés et un tel spectre d'action. Une certaine mise au point est régulièrement à faire pour tenir compte de l'évolution dudit spectre d'action.

La concentration d'antibiotique à mettre en oeuvre est calculée de manière à assurer au moins 5 fois la concentration minimale d'inhibition de la solution (CMI). Toutefois, la dose totale d'antibiotique injectée reste tout à fait négligeable, tout en assurant la stérilité de la solution.

Avantageusement, on peut employer un mélange tétracycline + érythromycine.

Les émulsions ainsi obtenues sont donc non-allergisantes et parfaitement stériles. Elles peuvent être soumises favorablement à divers contrôles bactériologiques.

Les extraits ainsi obtenus sont conservés par des techniques de froid qui permettent des contrôles biologiques, bactériologiques, virologiques et immunologiques pour prouver l'inocuité totale de ces extraits. Lesdits extraits sont avantageusement surgelés puis maintenus congelés.

Les émulsions obtenues par le procédé B ne doivent pas nécessairement être surgelées à l'azote liquide (-196°C). Pour ladite surgélation, on emploie avantageusement un mélange de carboglace et d'alcool méthylique (environ -80°C). Ensuite, les solutions selon l'invention peuvent être conservées dans un congélateur ordinaire (environ -35°C)

On a observé qu'après un tel traitement,- surgélation vers -80°C puis congélation vers -35°C, - les solutions selon l'invention peuvent être conservées pendant plus d'un an, sans altération. Les propriétés biologiques ne sont pas modifiées.

Avantageusement, les émulsions selon l'invention sont conditionnées avant leur surgélation, par exemple dans des flacons d'environ 5 cm³.

Selon le procédé C, la stérilité virale et bactériologique des extraits est obtenue par l'étape d'ultrafiltration. Ces extraits n'ont pas besoin d'être surgelés, ils peuvent être conservés à la température ambiante ou au réfrigérateur.

Les extraits tissulaires bruts selon l'invention peuvent être obtenus à partir de tout organe de l'embryon et notamment le placenta, le foie, la rate, l'hypothalamus, la thyroïde, la surrénale, les testicules, les ovaires, le mésenchyme, le thymus, le cerveau, la moelle épinière, la moelle osseuse, les yeux, le pancréas, les reins, les poumons, le myocarde, l'hypophyse ou leurs mélanges, le cordon ombilical et le liquide amniotique.

Deux ou plusieurs organes peuvent être associés pour la réalisation des extraits tissulaires embryonnaires selon l'invention.

Selon la présente invention, on a mis en évidence les propriétés thérapeutiques de tels extraits tissulaires embryonnaires d'organes animaux.

Leur administration stimule les fonctions de l'organe correspondant.

Des tests ont été réalisés in vitro et in vivo.

Ainsi, des extraits tissulaires embryonnaires de mésenchyme (particulièrement celui extrait au niveau des odontoblastes), obtenus selon le procédé B, doublent en 24 heures la multiplication d'une culture de chondroblastes.

Cette action in vitro sur la cellule cartilagineuse est confirmée in vivo par une action remarquable sur les processus dégénératifs arthrosiques, sur les parodontoses et les retards de consolidation osseuse.

De la même façon, on a mis en évidence l'action d'extraits de thymus selon l'invention. On a montré que le thymus est un important stimulateur de l'immunité. Injecté à des enfants présentant des affections ORL à répétition ou à des personnes sujettes à de fréquentes infections pulmonaires, des extraits de thymus selon l'invention ont permis d'éviter la survenue ultérieure de telles atteintes. Une injection 2 fois par an est le plus souvent suffisante.

Des extraits de thymus seul, ou de thymus et de rate, ont permis la régularisation de l'indice d'immunité du test de Vernes, chez de nombreux sujets atteints de divers carcinomes et de traiter la maladie de Steinert, où on a mis en évidence grâce au traitement, une diminution significative du taux sanguin des enzymes musculaires (CPK, etc...).

Les extraits selon l'invention préparés à partir de thymus apparaissent comme d'importants immunostimulants, sans effet secondaire. Ils seront donc avantageusement employés pour le traitement de toutes les maladies où l'immunité est déprimée (maladies virales, SIDA) seuls ou en association avec toute autre médication.

On a également mis en évidence que des extraits issus du mélange placenta, foie, rate, hypothalamus sont particulièrement actifs sur les différentes manifestations de l'arthéro-sclérose ; des extraits d'oeil ont une action remarquable sur la cataracte et les presbyties ; des extraits de surrénale agissent sur l'asthénie, l'hypotension orthostatique et sont en mesure de remonter des taux trop bas de cortisol plasmatique, des extraits de placenta ont une action cicatrisante, par exemple sur des escarres cutanées.

Avec un extrait de liquide amniotique préparé selon le procédé C, on a obtenu une augmentation significative du métabolisme de fibroblastes (cellules dermiques) en culture cellulaire. On a donc la preuve que cet extrait a des propriétés anti-rides et anti-vergetures. L'extrait de placenta et de thymus selon l'invention a une action sur les symptômes et les signes biologiques de l'arthrite rhumatoïde.

## Revendications

1. Extraits tissulaires embryonnaires d'organes animaux,. administrables à l'homme, caractérisés en ce qu'ils sont obtenus par dilution ou ultrafiltration d'un extrait brut préparé à partir d'organes, prélevés selon des techniques connues, sur des embryons avant la première moitié de la gestation, broyés dans du sérum physiologique puis filtrés.

2. Extraits tissulaires selon la revendication 1, caractérisés en ce que lesdits organes sont prélevés sur des mammifères et notamment des bovins ou des ovins.

3. Extraits tissulaires selon l'une des revendications 1 ou 2, caractérisés en ce que lesdits organes sont choisis parmi le placenta, le foie, la rate, l'hypothalamus, la thyroïde, la surrénale, les testicules, les ovaires, le mésenchyme, le thymus, le cerveau, la moelle épinière, la moelle osseuse, les yeux, le pancréas, les reins, les poumons, le myocarde, l'hypophyse ou leurs mélanges.

4. Extraits tissulaires selon l'une quelconque des revendications 1 à 3, caractérisés en ce qu'ils sont obtenus à partir du thymus, seul ou associé à la rate.

5. Extraits tissulaires selon l'une quelconque des revendications 1 à 4, caractérisés en ce qu'ils sont obtenus par dilution de l'extrait brut dans un sérum physiologique en présence d'un agent conservateur ou d'un antibiotique.

6. Extraits tissulaires selon la revendication 5, caractérisés en ce que la concentration d'antibiotique représente au moins 5 fois la concentration minimale d'inhibition.

7. Extraits tissulaires selon l'une des revendications 5 ou 6, caractérisés en ce que l'antibiotique est un mélange tétracycline + érythromycine.

8. Extraits tissulaires selon l'une quelconque des revendications 5 à 7, caractérisés en ce qu'ils sont conservés par congélation après surgélation.

9. Extraits tissulaires selon l'une quelconque des revendications 5 à 8, caractérisés en ce que la surgélation est réalisée dans un mélange de carboglace et d'alcool méthylique.

10. Extraits tissulaires selon l'une quelconque des revendications 1 à 4, caractérisés en ce qu'ils sont obtenus par ultrafiltration de l'extrait brut.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| Y | DE-A-3 524 794 (CHEMISCHES INSTITUT SCHÄFER AG) <br> * Revendications 1-8,10-12; page 21, lignes 14-30; exemples 1,3 * | 1-10 | A 61 K 35/54 |
| Y | US-A-4 548 813 (R.L. LAWSON) <br> * Revendications 1-3,5-6; colonne 3, lignes 23-63 * | 1-10 | |
| A | FR-A-2 413 912 (R. BONTEMPS) <br> * Revendications 1,8 * | 1-10 | |
| A | DE-A-2 819 131 (K. THEURER) <br> * En entier * | 1-10 | |
| P,X | FR-A-2 578 743 (R. BONTEMPS) <br><br> * Revendications; page 5, ligne 13 - page 6, ligne 10 * | 1-3,5-10 | **DOMAINES TECHNIQUES RECHERCHES (Int. Cl.4)** <br><br> A 61 K |

Le présent rapport de recherche a été établi pour toutes les revendications

| Lieu de la recherche <br> LA HAYE | Date d'achèvement de la recherche <br> 18-09-1987 | Examinateur <br> RYCKEBOSCH A.O.A. |
|---|---|---|

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

OEB Form 1503 03 82